Europäisches Patentamt

European Patent Office

Office européen des brevets

(11) Publication number: **0 154 513**
**B1**

# (12) EUROPEAN PATENT SPECIFICATION

(45) Date of publication of patent specification: 24.05.89

(51) Int. Cl.⁴: **C 03 C 3/097,** C 03 C 8/02,
C 23 D 5/00

(21) Application number: 85301355.5

(22) Date of filing: 28.02.85

(54) **Glass compositions for bonding to alloys.**

(30) Priority: 01.03.84 US 584976

(43) Date of publication of application:
11.09.85 Bulletin 85/37

(45) Publication of the grant of the patent:
24.05.89 Bulletin 89/21

(84) Designated Contracting States:
AT CH DE FR GB IT LI SE

(56) References cited:
DE-A-3 248 649
US-A-4 234 972

ENGINEERING IN MEDICINE, vol. 9, no. 4,
October 1980, pages 201-207, MEP Ltd.,
Whitstable, Kent, GB; M. SEMLITSCH:
"Properties of wrought CoNiCrMo alloy
Protasul-10, a highly corrosion and fatigue
resistant implant material for joint
endoprostheses"

(73) Proprietor: **Pfizer Hospital Products Group, Inc.**
**235 East 42nd Street**
**New York New York 10017 (US)**

(72) Inventor: **Greenspan, David Charles**
**9722 Water Oak Drive**
**Fairfax Virginia (US)**

(74) Representative: **Wood, David John et al**
**Pfizer Limited Ramsgate Road**
**Sandwich Kent CT13 9NJ (GB)**

Courier Press, Leamington Spa, England.

## Description

The present invention relates to novel glass compositions which are suitable for bonding to alloys, in particular "Vitallium" (Trade Mark) alloys.

The use of biologically active glass compositions for bonding to metal substrates is known in the art, e.g. U.S. Patent No. 4,234,972. The glass coated metal substrates are suitable for prostheses or surgical implants.

The glass composition may contain, by weight, 40—60% $SiO_2$, 10—32% $Na_2O$, 10—32% CaO, 0—12% $P_2O_5$, 0—18% $CaF_2$, and 0—20% $B_2O_3$. One of the compositions disclosed in the patent contains 40.0% $SiO_2$, 24.5% $Na_2O$, 24.5% CaO, 6.0% $P_2O_5$ and 5.0% $B_2O_3$. There is no close match between the coefficients of thermal expansion of the glass composition and the metal substrate thereby making application of the glass composition on the metal substrate by conventional techniques difficult.

US Patent 4497629, equivalent to patent document DE 3248649 discloses a dental implant comprising a biocompatible metal core and a multi-layer bioglass coating having the same thermal expansion coefficient as the core. The inner and outer glass layers contain 35—60 mole% $SiO_2$, 10—30% $Na_2O$, 5—40% CaO as essential constituents and the outer layer contains 5—10% of $TiO_2$ also, the content of $TiO_2$ of the inner layer being less.

The invention has as an object the development of a biologically active composition suitable for application by conventional enamel coating techniques. The compositions according to the invention generally fulfill the following requirements:

(1) They are biologically active meaning that upon reaction with body fluids they form a series of surface reactive films whereby living tissue will chemically bond to the glass. (2) They have a softening point low enough that they can flow onto a metallic substrate without compromising the mechanical and physical properties of the metal, e.g. below 600°C. (3) They have a thermal coefficient which essentially matches that of the metal substrate, e.g. Vitallium substrate, so that a matched glass to metal seal may be formed following standard glass enameling procedures. A thermal expansion coefficient difference of less than 2% is generally necessary to prevent unwanted stresses from developing in the glass or the glass/metal interface. (4) They further react with an adherent oxide film on the metal substrate to form a diffusional bond with the substrate.

According to the invention, there is provided a glass composition consisting of, in percentages by weight based on the total weight of the composition, the following ingredients:

| (a) | $SiO_2$ | 44.65 |
|---|---|---|
| | $Na_2O$ | 20.01 |
| | CaO | 19.59 |
| | $B_2O_3$ | 10.30 |
| | $P_2O_5$ | 5.45 |

or

| (b) | $SiO_2$ | 43.83 |
|---|---|---|
| | $Na_2O$ | 18.41 |
| | CaO | 21.63 |
| | $B_2O_3$ | 10.11 |
| | $P_2O_5$ | 6.02 |

or

| (c) | $SiO_2$ | 47.45 |
|---|---|---|
| | $Na_2O$ | 19.48 |
| | CaO | 21.60 |
| | $B_2O_3$ | 5.45 |
| | $P_2O_5$ | 6.02 |

and having a softening temperature of 573°C, 584°C or 569°C respectively and a thermal expansion coefficient of $13.43 \times 10^{-6}$/°C, $13.31 \times 10^{-6}$/°C or $13.12 \times 10^{-6}$/°C respectively.

The compositions of the invention satisfy the above requirements (1)—(4). The compositions are particularly suited for coating of cobalt-chromium-molybdenum alloys having the following composition by weight, according to ASTM:

| Element | Composition, % | |
|---|---|---|
| | Min. | Max. |
| Chromium | 27.0 | 30.0 |
| Molybdenum | 5.0 | 7.0 |
| Nickel | ... | 2.5 |
| Iron | ... | 0.75 |
| Carbon | ... | 0.35 |
| Silicon | ... | 1.00 |
| Manganese | ... | 1.0 |
| Cobalt | 68.0 | 57.4 |
| | (Max) | (Min) |

Vitallium alloy is the preferred alloy and consists of:

| Element | % by weight |
|---|---|
| Carbon | 0.25 |
| Silicon | 0.75 |
| Manganese | 0.70 |
| Chromium | 28.00 |
| Molybdenum | 5.50 |
| Cobalt | 64.80 |

The thermal expansion coefficient of cast Vitallium is $13.21 \times 10^{-6}$/°C.

The invention includes coated substrates comprising a metal substrate such as one made of a cobalt-chromium-molybdenum surgical implant alloy, e.g. Vitallium alloy, and a coating made of the biologically active glass composition of the invention. As discussed above, the metal has a thermal expansion coefficient (X) which is essentially the same as the X of said biologically active glass composition, that is these coefficients differ by less than 2%.

The glass compositions are made by usual methods for making glasses involving mixing of the ingredients, melting and then cooling.

The coating of the Co—Cr—Mo alloy with the biologically active glass compositions of the invention is done by conventional methods. The glass to be used is ground e.g. to 200 mesh size or smaller. The glass is formed into a slurry by thorough mixing with liquids such as ethanol or acetone. The alloy surface is cleaned, preferably ultrasonically in methanol, and dried, e.g. gently blow-dried with a particle-free aerosol. The alloy is oxidized in air at about 593°C cooled to room temperature and stored in a substantially moisture free enclosure. The above glass slurry is applied e.g. by an airbrush at a distance of 8.9 cm (3.5 inches) at 69 KPa (10 psi) and variable angle to assure uniform coating. After coating, the alloy is allowed to dry before firing. The firing is at elevated temperatures of about 538° to 704°C, preferably at least part of the time under vacuum, and followed by cooling to room temperature. Finally, annealing is done by heating to about 450°C for about 2.5 hours. After cooling to room temperature, the coated alloy is preferably stored under substantially moisture free conditions.

The three compositions of the invention (Examples I to III) satisfy the above four conditions. They have low softening points ($T_s$) of lower than 600°C and excellent biological activity. These three compositions have a thermal expansion coefficient which essentially matches that of cast Vitallium alloy.

Example 1

| Composition | % by weight |
|---|---|
| $SiO_2$ | 44.65 |
| $Na_2O$ | 20.01 |
| $CaO$ | 19.59 |
| $B_2O_3$ | 10.30 |
| $P_2O_5$ | 5.45 |

This composition has a softening temperature of about 573°C and a thermal expansion coefficient of $13.43 \times 10^{-6}/°C$.

Example 2

| Composition | % by weight |
|---|---|
| $SiO_2$ | 43.83 |
| $Na_2O$ | 18.41 |
| $CaO$ | 21.63 |
| $B_2O_3$ | 10.11 |
| $P_2O_5$ | 6.02 |

$T_s = 584°C$ and $\alpha = 13.31 \times 10^{-6}/°C$.

Example 3

| Composition | % by weight |
|---|---|
| $SiO_2$ | 47.45 |
| $Na_2O$ | 19.48 |
| $CaO$ | 21.60 |
| $B_2O_3$ | 5.45 |
| $P_2O_5$ | 6.02 |

$T_s = 569°C$
$\alpha = 13.12 \times 10^{-6}/°C$.

Tests have been conducted to show that the glass compositions of Examples 1—3 meet the four requirements mentioned above. The tests were also conducted with two biologically active compositions, Examples 4 and 5, different to the compositions of the invention, showing that they do not have a thermal coefficient of expansion matching that of Vitallium alloy.

Example 4 (comparison)

| Composition | % by weight |
|---|---|
| $SiO_2$ | 50.8 |
| $Na_2O$ | 21.6 |
| $CaO$ | 21.6 |
| $P_2O_5$ | 6.0 |

$\alpha = 14.32 \times 10^{-6}/°C$. $T_s = 540°C$.

Example 5 (comparison)

| | |
|---|---|
| $SiO_2$ | 48.59 |
| $Na_2O$ | 20.82 |
| $CaO$ | 19.57 |
| $B_2O_3$ | 5.58 |
| $P_2O_5$ | 5.45 |

$\alpha = 13.70 \times 10^{-6}/°C$. $T_s = 561°C$.

The tests were performed with samples prepared as follows:

Vitallium alloy pieces were sand blasted, cleaned, and then oxidized at 649°C under 3600 Pa (27 mm Hg) vacuum for 5 minutes to produce a thin, adherent oxide film. The glass compositions of Examples 1—5 were then applied to the metal surface as a slurry. The coated article was then heated gradually to 538°C in air. After a 45 minute soak, the sample was subjected to a vacuum of 3600 Pa (27 mm Hg) and heated to 593°C with a 5 minute soak. The vacuum was broken and the sample heated further to 704°C. When that temperature was reached, the sample was removed from the furnace and allowed to cool.

The samples thus produced were analyzed for mechanical integrity of the glass to metal bond. The compositions of Examples 1—3 showed excellent mechanical integrity whereas the glasses of Examples 4 and 5 fail to meet the mechanical integrity requirements.

**Claims**

1. A glass composition consisting of, in percentages by weight based on the total weight of the composition, the following ingredients:

| (a) | $SiO_2$ | 44.65 |
|---|---|---|
| | $Na_2O$ | 20.01 |
| | $CaO$ | 19.59 |
| | $B_2O_3$ | 10.30 |
| | $P_2O_5$ | 5.45 |

or

(b)  SiO$_2$  43.83
Na$_2$O  18.41
CaO  21.63
B$_2$O$_3$  10.11
P$_2$O$_5$  6.02

or

(c)  SiO$_2$  47.45
Na$_2$O  19.48
CaO  21.60
B$_2$O$_3$  5.45
P$_2$O$_5$  6.02

and having a softening temperature of 573°C, 584°C or 569°C respectively and a thermal expansion coefficient of $13.43\times10^{-6}/°C$, $13.31\times10^{-6}/°C$ or $13.12\times10^{-6}/°C$ respectively.

2. A coated substrate which comprises a metallic substrate and a coating made of glass composition as claimed in claim 1 characterised in that the metallic substrate is an alloy having a thermal expansion coefficient which differs from the thermal expansion coefficient of the glass composition by less than 2%.

3. A coated substrate according to claim 2 characterised in that said substrate is a surgical implant.

**Patentansprüche**

1. Glaszusammensetzung, die in.Gew.%, bezogen auf das Gesamtgewicht der Zusammensetzung, aus den folgenden Bestandteilen besteht:

(a)  SiO$_2$  44,65
Na$_2$O  20,01
CaO  19,59
B$_2$O$_3$  10,30
P$_2$O$_5$  5,45

oder

(b)  SiO$_2$  43,83
Na$_2$O  18,41
CaO  21,63
B$_2$O$_3$  10,11
P$_2$O$_5$  6,02

oder

(c)  SiO$_2$  47,45
Na$_2$O  19,48
CaO  21,60
B$_2$O$_3$  5,45
P$_2$O$_5$  6,02

und eine Erweichungstemperatur von 573, 584 bzw. 569°C und einen Wärmeausdehnungskoeffizienten von $13,43\times10^{-6}/°C$, $13,31\times10^{-6}/°C$ bzw. $13,12\times10^{-6}/°C$ aufweist.

2. Beschichtetes Substrat, welches ein Metallsubstrat und eine Beschichtung hergestellt aus der in Anspruch 1 beanspruchten Glaszusammensetzung umfaßt, dadurch gekennzeichnet, daß das Metallsubstrat eine Legierung mit einem Wärmeausdehnungskoeffizienten ist, der sich von Wärmeausdehnungskoeffizienten der Glaszusammensetzung um weniger als 2% unterscheidet.

3. Beschichtetes Substrat nach Anspruch 2, dadurch gekennzeichnet, daß dieses Substrat ein chirurgiskhes Implantat ist.

**Revendications**

1. Composition de verre renfermant les ingrédients suivants en pourcentage pondéral par rapport au poids total de la composition:

(a)  SiO$_2$  44,65
Na$_2$O  20,01
CaO  19,59
B$_2$O$_3$  10,30
P$_2$O$_5$  5,45

ou

(b)  SiO$_2$  43,83
Na$_2$O  18,41
CaO  21,63
B$_2$O$_3$  10,11
P$_2$O$_5$  6,02

ou

(c)  SiO$_2$  47,45
Na$_2$O  19,48
CaO  21,60
B$_2$O$_3$  5,45
P$_2$O$_5$  6,02

ayant respectivement une température de ramollissement de 573°C, 584°C ou 569°C et respectivement un coefficient de dilatation thermique de $13,43\times10^{-6}/°C$, $13,31\times10^{-6}/°C$ et $13,12\times10^{-6}/°C$.

2. Substrat revêtu formé d'un substrat métallique et d'un revêtement fait de la composition de verre selon la revendication 1, caractérisé en ce que le substrat métallique est un alliage ayant un coefficient de dilatation thermique qui diffère du coefficient de dilatation thermique de la composition de verre de moins de 2%.

3. Substrat revêtu selon la revendication 2, caractérisé en ce que ledit substrat est un implant chirurgical.